# EUROPEAN PATENT APPLICATION

(11) **EP 4 332 242 A1**
(43) Date of publication of application: **06.03.2024**
(21) Application number: 22796218.0
(22) Date of filing: 29.04.2022
(51) Int. Cl.: C12Q 1/6886, G01N 33/574

(54) **METHOD FOR PREDICTING PROGNOSIS OF GASTRIC CANCER**

(30) Priority: 29.04.2021 KR 20210055921
(71) Applicant: Industry-Academic Cooperation Foundation, Yonsei University, Seoul 03722 (KR)
(72) Inventor: CHEONG, Jae-Ho, Seoul 06628 (KR); KIM, Jae-woo, Seoul 06574 (KR); YOON, Bo Kyung, Seoul 04731 (KR)
(74) Representative: V.O.
(86) International application number: PCT/KR2022/006198
(87) International publication number: WO 2022/231386

(57) **Abstract**

A composition and method according to the present invention may be very effectively used to diagnose malignant gastric cancer and predict the prognosis of gastric cancer.

## Description

### Technical Field

The present invention relates to a method for diagnosing malignant gastric cancer and predicting the prognosis of gastric cancer.

### Background Art

Cancer is a very fatal disease that can threaten the life of an individual by causing tissue cells to proliferate abnormally and unlimitedly to form a tumor that prevents the organ from performing its normal function. In 2017, the first leading cause of death in Korea was malignant neoplasm (cancer), and 27.6% of the total deaths were due to cancer. In particular, gastric cancer (GC) is the third most common fatal cancer in the world. Although there are differences in the site of cancer occurrence depending on race, gender, and region, recent molecular genomic technology has been able to classify gastric cancer types according to molecular characteristics.

Among biologically relevant subtypes of gastric cancer, especially those with stem-like properties are difficult to treat due to their malignant biological characteristics and have the worst prognosis due to unresponsiveness to standard chemotherapy. In addition, immune checkpoint-blockade therapy has also been confirmed to be ineffective against stem-like/EMT cancer subtypes. Therefore, there is a problem in that targeted therapy cannot be applied to these subtypes.

Accordingly, the present inventors have studied the molecular and metabolic characteristics of stem-like subtype gastric cancer (GC) and developed a method capable of effectively diagnosing malignant gastric cancer.

### DISCLOSURE

### Technical Problem

An object of the present invention is to provide a composition for predicting the prognosis of gastric cancer.

Another object of the present invention is to provide a kit for predicting the prognosis of gastric cancer patients.

Still another object of the present invention is to provide a method of providing information for predicting the prognosis of gastric cancer patients.

Yet another object of the present invention is to provide a diagnostic system for predicting the prognosis of gastric cancer patients.

However, objects to be achieved by the present invention are not limited to the objects mentioned above, and other objects not mentioned herein can be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the art to which the present invention pertains.

One embodiment of the present invention is directed to a composition for predicting the prognosis of cancer.

In the present invention, the composition may contain an agent for measuring the expression level of at least one gene selected from the group consisting of TAGLN, CALD1, MYL9, MYLK, ACTA2, TPM2, CNN1, MSN, CNN3, MYH10, MYH11, CRIP2, and ITGA5, or a protein encoded thereby.

In the present invention, "transgelin (TAGLN)" refers to a protein encoded by the TAGLN gene in humans. The protein encoded by this gene is known as a transformation and shape-change sensitive actin cross-linking/gelling protein found in fibroblasts and smooth muscles. Information on the TAGLN protein and gene is registered with NCBI (National Center for Biotechnology Information) (Gene ID: 6876), and the amino acid sequence of the TAGLN protein is set forth in SEQ ID NO: 1. In the present invention, the TAGLN protein or the gene encoding the same is of human origin, but species from which the TAGLN protein or the gene encoding the same may originate include various species without being limited to humans.

In the present invention, "caldesmon 1 (CALD1)" refers to a calmodulin binding protein encoded by the CALD1 gene in humans. Like calponin, caldesmon is also known to inhibit the ATPase activity of myosin in smooth muscle. Information on the CALD1 protein and gene is registered with NCBI (National Center for Biotechnology Information) (Gene ID: 800), and the amino acid sequence of the CALD1 protein is set forth in SEQ ID NO: 2. In the present invention, the CALD1 protein or the gene encoding the same is of human origin, but species from which the CALD1 protein or the gene encoding the same may originate include various species without being limited to humans.

In the present invention, "myosin light chain 9 (MYL9)" refers to a protein encoded by the MYL9 gene in humans. Myosin, a structural component of muscle, consists of two heavy chains and four light chains, and the protein encoded by this gene is known to be a myosin light chain that can regulate muscle contraction by regulating the ATPase activity of the myosin head. Information on the MYL9 protein and gene is registered with NCBI (National Center for Biotechnology Information) (Gene ID: 10398), and the amino acid sequence of the MYL9 protein is set forth in SEQ ID NO: 3. In the present invention, the MYL9 protein or the gene encoding the same is of human origin, but species from which the MYL9 protein or the gene encoding the same may originate include various species without being limited to humans.

In the present invention, "myosin light chain kinase (MYLK)" refers to an enzyme encoded by the MYLK gene in humans. This gene, a muscle member of the immunoglobulin superfamily, encodes myosin light chain kinase, a calcium/calmodulin dependent-enzyme. The kinase is also known to phosphorylate the myosin regulatory light chain and facilitate myosin interaction with actin filaments to produce contractile activity. Information on the MYLK protein and gene is registered with NCBI (National Center for Biotechnology Information) (Gene ID: 4638), and the amino acid sequence of the MYLK protein is set forth in SEQ ID NO: 4. In the present invention, the MYLK protein or the gene encoding the same is of human origin, but species from which the MYLK protein or the gene encoding the same may originate include various species without being limited to humans.

In the present invention, "actin alpha 2 (ACTA2)" refers to a protein encoded by the ACTA2 gene in humans. Actin is a family of globular multi-functional proteins that form microfilaments. ACTA2 is one of the six actin isoforms and is also known to be involved in the contractile apparatus of smooth muscles. Information on the ACTA2 protein and gene is registered with NCBI (National Center for Biotechnology Information) (Gene ID: 59), and the amino acid sequence of the ACTA2 protein is set forth in SEQ ID NO: 5. In the present invention, the ACTA2 protein or the gene encoding the same is of human origin, but species from which the ACTA2 protein or the gene encoding the same may originate include various species without being limited to humans.

In the present invention, "tropomyosin 2 (TPM2)" is also called beta-tropomyosin (β-tropomyosin) and refers to a protein encoded by the TPM2 gene in humans. TPM2 is known to function to stabilize actin filaments and control muscle contraction. Information on the TPM2 protein and gene is registered with NCBI (National Center for Biotechnology Information) (Gene ID: 7169), and the amino acid sequence of the TPM2 protein is set forth in SEQ ID NO: 6. In the present invention, the TPM2 protein or the gene encoding the same is of human origin, but species from which the TPM2 protein or the gene encoding the same may originate include various species without being limited to humans.

In the present invention, "calponin 1 (CNN1)" refers to a smooth muscle protein encoded by the CNN1 gene in humans. CNN1 is located at 19p13.2-p13.1 in the human chromosomal genome and is known to contain seven exons encoding calponin 1, an actin filament-related regulatory protein. Information on the CNN1 protein and gene is registered with NCBI (National Center for Biotechnology Information) (Gene ID: 1264), and the amino acid sequence of the CNN1 protein is set forth in SEQ ID NO: 7. In the present invention, the CNN1 protein or the gene encoding the same is of human origin, but species from which the CNN1 protein or the gene encoding the same may originate include various species without being limited to humans.

In the present invention, "moesin (MSN)" refers to a protein encoded by the MSN gene in humans. MSN is known to be a member of the ERM protein family that functions as a cross-linker between the plasma membrane and the actin-based cytoskeleton. Information on the MSN protein and gene is registered with NCBI (National Center for Biotechnology Information) (Gene ID: 4478), and the amino acid sequence of the MSN protein is set forth in SEQ ID NO: 8. In the present invention, the MSN protein or the gene encoding the same is of human origin, but species from which the MSN protein or the gene encoding the same may originate include various species without being limited to humans.

In the present invention, "calponin 3 (CNN3)" refers to a protein encoded by the CNN3 gene in humans. CNN3 is known to be located at 1p22-p21 in the human chromosomal genome. Information on the CNN3 protein and gene is registered with NCBI (National Center for Biotechnology Information) (Gene ID: 1266), and the amino acid sequence of the CNN3 protein is set forth in SEQ ID NO: 9. In the present invention, the CNN3 protein or the gene encoding the same is of human origin, but species from which the CNN3 protein or the gene encoding the same may originate include various species without being limited to humans.

In the present invention, "myosin heavy chain 10 (MYH10)" is also called non-muscle myosin IIB (NM-IIB) and refers to a protein encoded by the MYH10 gene in humans. NM-IIB is part of the myosin II subfamily of proteins, which also includes skeletal muscle, cardiac muscle and smooth muscle myosins. NM-IIB, and non-muscle myosins in general, are known to be widely expressed in every tissue in humans. Information on the MYH10 protein and gene is registered with NCBI (National Center for Biotechnology Information) (Gene ID: 4628), and the amino acid sequence of MYH10 protein is set forth in SEQ ID NO: 10. In the present invention, the MYH10 protein or the gene encoding the same is of human origin, but species from which the MYH10 protein or the gene encoding the same may originate include various species without being limited to humans.

In the present invention, "myosin heavy chain 11 (MYH11)" refers to a protein encoded by the MYH11 gene in humans. MYH11 is a smooth muscle myosin belonging to the myosin heavy chain family and is also known to be a major contractile protein that converts chemical energy into mechanical energy through the hydrolysis of ATP. Information on the MYH11 protein and gene is registered with NCBI (National Center for Biotechnology Information) (Gene ID: 4629), and the amino acid sequence of the MYH11 protein is set forth in SEQ ID NO: 11. In the present invention, the MYH11 protein or the gene encoding the same is of human origin, but species from which the MYH11 protein or the gene encoding the same may originate include various species without being limited to humans.

In the present invention, "cysteine rich protein 2 (CRIP2)" refers to a protein encoded by the CRIP2 gene in humans. CRIP2 is also known to be a protein containing two LIM domains. Information on the CRIP2 protein and gene is registered with NCBI (National Center for Biotechnology Information) (Gene ID: 1397), and the amino acid sequence of the CRIP2 protein is set forth in SEQ ID NO: 12. In the present invention, the CRIP2 protein or the gene encoding the same is of human origin, but species from which the CRIP2 protein or the gene encoding the same may originate include various species without being limited to humans.

In the present invention, "integrin subunit alpha 5 (ITGA5)" refers to a protein encoded by the ITGA5 gene in humans. The product of the gene belongs to the integrin-alpha chain family and is known to undergo post-translational cleavage in the extracellular domain to yield disulfide-linked light and heavy chains that join with beta 1 to form a fibronectin receptor. Information on the ITGA5 protein and gene is registered with NCBI (National Center for Biotechnology Information) (Gene ID: 3678), and the amino acid sequence of the ITGA5 protein is set forth in SEQ ID NO: 13. In the present invention, the ITGA5 protein or the gene encoding the same is of human origin, but species from which the ITGA5 protein or the gene encoding the same may originate include various species without being limited to humans.

In the present invention, the "prognosis" refers to the action of predicting the course of a disease and the outcome of death or survival. The "prognosis" or "predicting the prognosis" may be interpreted to refer to any action that predicts the course of the disease before and after treatment by comprehensively considering the physiological or environmental condition of the patient because the course of the disease may vary depending on this condition of the patient. For the purposes of the present invention, "predicting the prognosis" may be performed by predicting whether cancer stem cells are contained, and thus it may be interpreted as an action of predicting the course of cancer treatment and the survival rate or survival time of cancer patients in advance.

In the composition of the present invention, a subject whose prognosis is to be predicted may be a subject of interest that has or is highly likely to develop cancer. In the present invention, the "subject of interest" refers to mammals including humans. For example, the subject of interest may be selected from the group consisting of humans, rats, mice, guinea pigs, hamsters, rabbits, monkeys, dogs, cats, cows, horses, pigs, sheep, and goats. Preferably, the subject of interest may be a human, without being limited thereto.

In the present invention, the "human" refers to a person who has cancer or is suspected of having cancer, and means a patient who is in need of proper treatment of cancer or is expected to be in need of this treatment, without being limited thereto.

In the present invention, as the disease whose prognosis is to be predicted, the "cancer" refers to or describes the physiological condition typically characterized by unregulated cell growth in a mammal. The cancer may be gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma, but is not limited thereto and may be any type of cancer whose progression (such as tumor differentiation and/or proliferation) is dependent on the cancer cells or cancer stem cells described in the present invention. Preferably, the cancer may be gastric cancer, without being limited thereto.

When the composition of the present invention is used, it is possible to classify gastric cancer into subtypes, for example, mixed stroma subtype, gastric subtype, stem-like subtype, intestinal subtype, and inflammatory subtype, and in particular, it is possible to diagnose whether gastric cancer corresponds to the stem-like subtype, which has the worst prognosis.

In the composition of the present invention, the agent for measuring the expression level of the protein may comprise at least one selected from the group consisting of antibodies, oligopeptides, ligands, peptide nucleic acids (PNAs), and aptamers, which bind specifically to the protein, without being limited thereto.

In the present invention, the "antibody" refers to a substance that specifically binds to an antigen and causes an antigen-antibody reaction. For the purposes of the present invention, the antibody refers to an antibody that specifically binds to the protein. Examples of the antibody of the present invention include all of polyclonal antibodies, monoclonal antibodies, and recombinant antibodies. This antibody may be readily produced using techniques well known in the art. For example, a polyclonal antibody may be produced by a method well known in the art, which includes a process of obtaining a serum containing the antibody by injecting the antigen of the protein into an animal and collecting blood from the animal. This polyclonal antibody may be produced from any animal such as goat, rabbit, sheep, monkey, horse, pig, cow, dog, or the like. In addition, a monoclonal antibody may be produced using the hybridoma method well known in the art (see Kohler and Milstein (1976) European Journal of Immunology 6:511-519), or the phage antibody library technology (see Clackson et al, Nature, 352:624-628, 1991; Marks et al, J. Mol. Biol., 222:58, 1-597, 1991). The antibody produced by the above method may be isolated and purified using a method such as gel electrophoresis, dialysis, salt precipitation, ion exchange chromatography, or affinity chromatography. In addition, examples of the antibody of the present invention include not only a complete form having two full-length light chains and two full-length heavy chains, but also functional fragments of an antibody molecule. "Functional fragment of an antibody molecule" refers to a fragment having at least an antigen-binding function, and examples thereof include Fab, F(ab'), F(ab')2 and Fv.

In the present invention, the "oligopeptide" is a peptide consisting of 2 to 20 amino acids, and examples thereof include, but are not limited to, dipeptides, tripeptides, tetrapeptides, and pentapeptides.

In the present invention, the "peptide nucleic acid (PNA)" refers to an artificially synthesized DNA or RNA-like polymer, which was first introduced by the Professors Nielsen, Egholm, Berg and Buchardt at University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and significantly increased stability. Thus, PNA is used for molecular biology, diagnostic assays and antisense therapies. The PNA is disclosed in detail in the literature [Nielsen P E, Egholm M, Berg R H, Buchardt O (December 1991). "Sequence-selective recognition of DNA by strand displacement with a thymine-substituted polyamide". Science 254(5037): 1497-1500].

In the present invention, the "aptamer" refers to an oligonucleotide or a peptide molecule, and the general contents of the aptamer are disclosed in detail in the literature [Bock L C et al., Nature 355(6360):5646(1992); Hoppe-Seyler F, Butz K "Peptide aptamers: powerful new tools for molecular medicine". J Mol Med. 78(8):42630(2000); Cohen B A, Colas P, Brent R. "An artificial cell-cycle inhibitor isolated from a combinatorial library". Proc Natl Acad Sci USA. 95(24): 142727(1998)].

In composition of the present invention, the agent for measuring the expression level of the gene encoding the protein may comprise at least one selected from the group consisting of primers, probes, and antisense nucleotides, which bind specifically to the gene encoding the protein, without being limited thereto.

In the present invention, the "primer" refers to a fragment that recognizes a target gene sequence, and comprises a pair of forward and reverse primers, but is preferably a pair of primers providing analysis results with specificity and sensitivity. When the nucleotide sequence of the primer is a sequence inconsistent with the non-target sequence present in a sample, and thus is a primer that amplifies only the target gene sequence containing the complementary primer binding site without inducing non-specific amplification, high specificity may be imparted.

In the present invention, the "probe" refers to a substance capable of binding specifically to a target substance to be detected in a sample, and refers to a substance capable of specifically detecting the presence of the target substance in the sample through the binding. The type of probe is not particularly limited so long as it is commonly used in the art. Preferably, the probe may be PNA (peptide nucleic acid), LNA (locked nucleic acid), a peptide, a polypeptide, a protein, RNA, or DNA. Most preferably, the probe may be PNA. More specifically, the probe is a biomolecule derived from an organism or an analogue thereof, or is produced *in vitro.* Examples of the probe include an enzyme, a protein, an antibody, a microorganism, an animal and/or plant cell and organ, a neuron, DNA, and RNA. Examples of the DNA include cDNA, genomic DNA, and an oligonucleotide, examples of the RNA include genomic RNA, mRNA, and an oligonucleotide, and examples of the protein include antibodies, antigens, enzymes, peptides, and the like.

In the present invention, the "LNA (locked nucleic acid)" refers to a nucleic acid analogue containing a 2'-O or 4'-C methylene bridge [J Weiler, J Hunziker and J Hall Gene Therapy (2006) 13, 496.502]. LNA nucleosides comprise the common bases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand.

In the present invention, the term "antisense" means an oligomer that has a nucleotide sequence and a backbone between subunits, wherein an antisense oligomer is hybridized with the target sequence in the RNA by Watson-Crick base pairing to typically allow the formation of the mRNA and RNA: oligomer heterodimers in the target sequence. The oligomer may have an accurate or approximate sequence complementarity to the target sequence.

Information on the gene selected from the group consisting of TAGLN, CALD1, MYL9, MYLK, ACTA2, TPM2, CNN1, MSN, CNN3, MYH10, MYH11, CRIP2 and ITGA5, or the protein encoded by the gene according to the present invention is known. Thus, based on this information, any person skilled in the art will be able to easily design a primer, probe or antisense nucleotide that binds specifically to the gene.

Another embodiment of the present invention is directed to a kit for predicting the prognosis of cancer comprising the composition of the present invention.

In the present invention, the "kit" refers to a tool in which a probe or antibody that binds specifically to a biomarker component is labeled with a detectable substance so that the expression level of the biomarker may be assessed. The term of labeled, with regard to the probe or antibody, is intended to encompass direct labeling of the probe or antibody by the reaction of a detectable substance with a substrate, as well as indirect labeling of the probe or antibody by reactivity with another reagent that is directly labeled. The kit may comprise a chromogenic substrate solution to induce a chromogenic reaction with the label, a washing liquid, and other solutions, and may be prepared to comprise reagent components used. In the present invention, the kit may be a kit comprising essential components necessary for performing RT-PCR, and may comprise, in addition to each primer pair specific for the marker gene, a test tube, a reaction buffer, deoxynucleotides (dNTPs), Taq-polymerase, reverse transcriptase, DNase and RNase inhibitors, sterile water, and the like. The kit may also be a kit for detecting the gene for predicting cancer prognosis and may comprise essential components necessary for performing DNA chip assay. The DNA chip kit may comprise a substrate to which a cDNA corresponding to a gene or a fragment thereof is attached as a probe, wherein the substrate may comprise a cDNA corresponding to a quantitative control gene or a fragment thereof. The kit of the present invention is not limited thereto and may be any type of kit known in the art.

In the present invention, the kit may be an RT-PCR kit, a DNA chip kit, an ELISA kit, a protein chip kit, a rapid kit, or a multiple reaction monitoring (MRM) kit.

The kit of the present invention may further comprise a composition, a solution or a device, which contains one or more different components suitable for the analysis method. For example, the kit in the present invention may further comprise essential components required for performing reverse transcription polymerase reaction. The reverse transcription polymerase reaction kit comprises a primer pair specific to the gene encoding the marker protein. The primer is an oligonucleotide having a sequence specific to the nucleotide sequence of the gene and may be about 7 to 50 bp in length, preferably about 10 to 30 bp in length. In addition, the kit may comprise a primer specific to the nucleotide sequence of a control gene. In addition, the reverse transcription polymerase reaction kit may comprise a test tube or other appropriate container, a reaction buffer (various pHs and magnesium concentrations), deoxynucleotides (dNTPs), enzymes such as Taq-polymerase and reverse transcriptase, DNAse and RNAse inhibitors, DEPC-water, sterilized water, etc.

In addition, the kit of the present invention may comprise essential components required for performing DNA chip assay. The DNA chip kit may comprise a substrate having immobilized thereon a cDNA or oligonucleotide corresponding to each gene or a fragment thereof, a reagent for constructing a fluorescence-labeled probe, an agent, an enzyme, and the like. In addition, the substrate may comprise a cDNA or oligonucleotide corresponding to a control gene or a fragment thereof.

In addition, the kit of the present invention may comprise essential components required for performing ELISA. The ELISA kit comprises an antibody specific to the protein. The antibody is a monoclonal antibody, a polyclonal antibody or a recombinant antibody, which has a high specificity and affinity for the marker protein and shows little or no cross-reactivity with other proteins. Also, the ELISA kit may comprise an antibody specific to a control protein. In addition, the ELISA kit may comprise reagents capable of detecting bound antibodies, for example, labelled secondary antibodies, chromophores, enzymes (e.g., conjugated with antibodies) and the substrates thereof or other substances which are capable of binding to antibodies.

In the kit of the present invention, a support for the antigen-antibody binding reaction may be selected from among nitrocellulose membranes, PVDF membranes, well plates made of polyvinyl or polystyrene resin, and slide glasses, without being limited thereto.

In addition, in the kit of the present invention, the secondary antibody is preferably labeled with a conventional chromogenic agent that participates in a chromogenic reaction. The chromogenic agent may be selected from among fluoresceins and dyes, such as horseradish peroxidase (HRP), alkaline phosphatase, colloidal gold, poly L-lysine-fluorescein isothiocyanate (FITC), and rhodamine-B-isothiocyanate (RITC), without being limited thereto.

In addition, in the kit of the present invention, a chromogenic substrate for inducing color development is preferably used depending on the label that participates in the chromogenic reaction. The chromogenic substrate may be selected from among 3,3',5,5'-tetramethylbenzidine (TMB), 2,2'-azino-bis(3-ethylbenzothiazoline)-6-sulfonic acid (ABTS), o-phenylenediamine (OPD), and the like. In this case, the chromogenic substrate is more preferably provided in a state in which it is dissolved in a buffer solution (0.1 M NaAc, pH 5.5). A chromogenic substrate such as TMB is degraded by HRP used as a label for the secondary antibody conjugate to generate a chromogenic deposit, and the deposition level of the chromogenic deposit may be checked visually, thereby detecting the presence or absence of the marker proteins.

In the kit of the present invention, the washing solution preferably contains a phosphate buffer solution, NaCl, and Tween 20, and is more preferably a buffer solution (PBST) composed of a 0.02 M phosphate buffer solution, 0.13 M NaCl, and 0.05% Tween 20. After the secondary antibody is allowed to react with the antigen-antibody conjugate after the antigen-antibody binding reaction, the support is washed 3 to 6 times with a proper amount of the washing solution. A sulfuric acid (H₂SO₄) solution is preferably used as a reaction stop solution.

In the kit of the present invention, details regarding the cancer and the at least one gene selected from the group consisting of TAGLN, CALD1, MYL9, MYLK, ACTA2, TPM2, CNN1, MSN, CNN3, MYH10, MYH11, CRIP2 and ITGA5 or the protein encoded thereby overlap with those described above with respect to the composition for predicting the prognosis of cancer according to the present invention, and thus detailed description thereof will be omitted.

Still another embodiment of the present invention is directed to a method of providing information for predicting the prognosis of cancer.

The method of the present invention may comprise a step of measuring the expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a biological sample isolated from a subject of interest.

The method of the present invention may be used to determine whether, in the biological sample isolated from the subject of interest, the prognosis of cancer is poor or is highly likely to be poor.

In the present invention, the subject of interest may be a subject that has cancer or is highly likely to develop cancer, and refers to mammals including humans. For example, the subject of interest may be selected from the group consisting of humans, rats, mice, guinea pigs, hamsters, rabbits, monkeys, dogs, cats, cows, horses, pigs, sheep, and goats, and is preferably a human, without being limited thereto.

In the present invention, the biological sample refers to any material, biological body fluid, tissue or cell obtained or derived from the subject. Specifically, the biological sample may be at least one selected from the group consisting of whole blood, leukocytes, peripheral blood mononuclear cells, buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, and cerebrospinal fluid, without being limited thereto.

In the present invention, the agent for measuring the expression level of the protein may comprise at least one selected from the group consisting of antibodies, oligopeptides, ligands, peptide nucleic acids (PNAs), and aptamers, which bind specifically to the protein.

In the present invention, the measurement of the expression level of the protein may be performed by protein chip assay, immunoassay, ligand binding assay, MALDI-TOF (Matrix Assisted Laser Desorption/Ionization Time of Flight Mass Spectrometry), SELDI-TOF (Surface Enhanced Laser Desorption/Ionization Time of Flight Mass Spectrometry), radioimmunoassay, radial immunodiffusion, Ouchterlony immunodiffusion, Rocket immunoelectrophoresis, immunohistostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

In addition, in the present invention, the measurement of the expression level of the protein may be performed by a multiple reaction monitoring (MRM) method.

In the present invention, the internal standard that is used in the multiple reaction monitoring method may be a synthetic peptide, obtained by substituting a specific amino acid of the target peptide with an isotope, or *Escherichia coli* beta-galactosidase.

In the present invention, the TAGLN protein may consist of the amino acid sequence set forth in SEQ ID NO: 1, without being limited thereto.

In the present invention, the CALD1 protein may consist of the amino acid sequence set forth in SEQ ID NO: 2, without being limited thereto.

In the present invention, the MYL9 protein may consist of the amino acid sequence set forth in SEQ ID NO: 3, without being limited thereto.

In the present invention, the MYLK protein may consist of the amino acid sequence set forth in SEQ ID NO: 4, without being limited thereto.

In the present invention, the ACTA2 protein may consist of the amino acid sequence set forth in SEQ ID NO: 5, without being limited thereto.

In the present invention, the TPM2 protein may consist of the amino acid sequence set forth in SEQ ID NO: 6, without being limited thereto.

In the present invention, the CNN1 protein may consist of the amino acid sequence set forth in SEQ ID NO: 7, without being limited thereto.

In the present invention, the MSN protein may consist of the amino acid sequence set forth in SEQ ID NO: 8, without being limited thereto.

In the present invention, the CNN3 protein may consist of the amino acid sequence set forth in SEQ ID NO: 9, without being limited thereto.

In the present invention, the MYH10 protein may consist of the amino acid sequence set forth in SEQ ID NO: 10, without being limited thereto.

In the present invention, the MYH11 protein may consist of the amino acid sequence set forth in SEQ ID NO: 11, without being limited thereto.

In the present invention, the CRIP2 protein may consist of the amino acid sequence set forth in SEQ ID NO: 12, without being limited thereto.

In the present invention, the ITGA5 protein may consist of the amino acid sequence set forth in SEQ ID NO: 13, without being limited thereto.

In the present invention, the expression level of the TAGLN, CALD1, MYL9, MYLK, ACTA2, TPM2, CNN1, MSN, CNN3, MYH10, MYH11, CRIP2 and ITGA5 genes or the protein(s) encoded thereby may be measured.

In the present invention, the agent for measuring the expression level of the gene encoding the protein may comprise at least one selected from the group consisting of primers, probes, and antisense nucleotides, which bind specifically to the gene encoding the protein.

In the present invention, the measurement of the expression level of the gene encoding the protein may be performed by reverse transcription-polymerase chain reaction (RT-PCR), competitive RT-PCR, real-time RT-PCR, RNase protection assay (RPA), Northern blotting, or DNA chip assay.

In the method of providing information according to the present invention, details regarding the antibodies, oligopeptides, ligands, peptide nucleic acids (PNA), aptamers, etc., and details regarding the primers, probes, etc. overlap with those described above, and thus detailed description thereof will be omitted to avoid excessive complexity of the specification.

In addition, in the present invention, when the expression level of at least one gene selected from the group consisting of TAGLN, CALD1, MYL9, MYLK, ACTA2, TPM2, CNN1, MSN, CNN3, MYH10, MYH11, CRIP2 and ITGA5, or the protein encoded thereby, measured in the biological sample isolated from the subject of interest, is higher than a control, it may be predicted that the subject of interest has developed or is highly likely to develop malignant cancer with stem cell properties, indicating that the prognosis of cancer treatment will be poor.

In the present invention, the "control" may be average or median expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a normal control group with no cancer or in cancer cells with no stem cell properties. The expression level of the marker protein or the nucleic acid molecule encoding the same in the cancer patient-derived biological sample to be analyzed may be compared with the expression level of the marker protein or the nucleic acid molecule encoding the same in the control group, whether malignant cancer has occurred or is likely to occur may be diagnosed by determining whether there is a significant change in the expression level. The scope of the normal control sample includes cells derived from cancer patients, cultures thereof, blood, serum, plasma, and tissues, which have been confirmed to have no stem cell properties.

In the present invention, the cancer may be gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma, but is not limited thereto and may be any type of cancer whose progression (such as tumor differentiation and/or proliferation) is dependent on the cancer cells or cancer stem cells described in the present invention. Preferably, the cancer may be gastric cancer, without being limited thereto.

In the present invention, the method may further comprise a step of calculating a smooth muscle cell (SMC) score from the measured expression level of the gene or the protein encoded thereby. The SMC score of the present invention may be processed using the TopHat-Cufflink 14 pipeline to determine normalized gene expression levels, without being limited thereto. When data are processed using the pipeline function, read counts can be obtained through a quantile normalization method. The calculation of the SMC score may be performed according to the edgeR user's guide using the EdgeR package, one of the most commonly used R packages among statistical systems commonly used in the art (http://www.bioconductor.org/packages/release/bioc/vignettes/edgeR/inst/doc/edgeRUse rsGuide.pdf). According to the above guide, the expression level of the gene or protein may be transformed into a log-intensity value from generalized linear models.

In the present invention, the SMC score may be calculated as the average value of the expression levels (preferably log intensities) of at least two genes selected from the group consisting of TAGLN, CALD1, MYL9, MYLK, ACTA2, TPM2, CNN1, MSN, CNN3, MYH10, MYH11, CRIP2 and ITGA5, or proteins encoded thereby. More preferably, the SMC score may be calculated as the average value of the log intensities of the expression levels of the two genes.

However, in the present invention, the expression level may be a value normalized by a housekeeping gene or a protein encoded thereby, without being limited thereto. Here, examples of the housekeeping gene include, but are not limited to, β-actin, AHSP, B2M, TUBB2a, GAPDH, HBS1L, HPRT1, and SDHA.

In one example of the present invention, the method for predicting the prognosis of cancer may further comprise a step of predicting that, when the calculated SMC score is 5 to 15 or more, preferably 10 to 12 or more, more preferably 11 or more, malignant cancer containing cancer stem cells has occurred or is highly likely to occur, and thus the treatment prognosis of the cancer patients is poor, or the survival rate of the cancer patient is low, or the survival time of the cancer patient is short.

In another example of the present invention, the method for predicting the prognosis of cancer according to the present invention may further comprise a step of predicting that, when the calculated SMC score is less than 5 to 15, preferably less than 10 to 12, more preferably less than 11, the treatment prognosis of the cancer patients is good, or the survival rate of the cancer patient is high, or the survival time of the cancer patient is long.

In the method of the present invention, details regarding the cancer and the at least one gene selected from the group consisting of TAGLN, CALD1, MYL9, MYLK, ACTA2, TPM2, CNN1, MSN, CNN3, MYH10, MYH11, CRIP2 and ITGA5 or the protein encoded thereby overlap with those described above with respect to the composition for predicting the prognosis of cancer according to the present invention, and thus detailed description thereof will be omitted.

Yet another embodiment of the present invention is directed to a diagnostic system for predicting the prognosis of cancer
The diagnostic system of the present invention may comprise: a measurement unit configured to measure the expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a biological sample isolated from a subject of interest; and a calculation unit configured to calculate a smooth muscle cell (SMC) score from the expression level of the gene or the protein encoded thereby, measured in the measurement unit.

The calculation unit of the diagnostic system of the present invention may perform the function of calculating the SMC score as the average of log intensities of the expression levels of the genes or proteins, measured in the measurement unit.

The treatment prognosis of cancer in the subject of interest may be predicted from the SMC score calculated in the calculation unit of the diagnostic system of the present invention. More specifically, the calculation unit may generate and classify information on cancer prognosis prediction according to the calculated SMC score, thereby determining that the subject of interest has a poor prognosis due to a malignant cancer with stem cell properties.

In one example of the present invention, the calculation unit may predict that, when the calculated SMC score is 5 to 15 or more, preferably 10 to 12 or more, more preferably 11 or more, malignant cancer containing cancer stem cells has occurred or is highly likely to occur, and thus the treatment prognosis of the cancer patient is poor, and the diagnostic system may further comprise an output unit configured to output the prediction result.

In another example of the present invention, the calculation unit may predict that, when the calculated SMC score is less than 5 to 15, preferably less than 10 to 12, more preferably less than 11, the treatment prognosis of the cancer patients is good, and the output unit may output the prediction result.

The diagnostic system of the present invention may further comprise an output unit configured to output the prognosis of cancer in the subject of interest, predicted in the calculation unit.

In the diagnostic system of the present invention, details regarding the cancer and the at least one gene selected from the group consisting of TAGLN, CALD1, MYL9, MYLK, ACTA2, TPM2, CNN1, MSN, CNN3, MYH10, MYH11, CRIP2 and ITGA5 or the protein encoded thereby overlap with those described above with respect to the composition for predicting the prognosis of cancer according to the present invention, and thus detailed description thereof will be omitted.

### Advantageous Effects

When the composition and method according to the present invention are used, it is possible to predict the prognosis of gastric cancer by diagnosing malignant cancer, especially malignant gastric cancer.

### Brief Description of Drawings

FIG. 1 shows the results of analyzing transcriptome data for tumors of gastric cancer patients (n = 497) in the Yonsei cohort according to one example of the present invention.
FIG. 2 shows the survival probability and gene set enrichment analysis (GSEA) results of stem-like GC subtype and intestinal GC subtype patients according to one example of the present invention.
FIG. 3a shows the results of converting SFRP4 microarray data into log values according to one example of the present invention.
FIG. 3b shows a heatmap of EMT signature genes in an SMC-low patient group and an SMC-high patient group according to one example of the present invention.
FIG. 4a shows the results of examining SFRP4 co-expression and the co-expression score of SMC genes constituting SMC score in gastric cancer according to one example of the present invention.
FIG. 4b shows the results of examining the SMC score versus SFRP4 in the gastric cancer data set (The Cancer Genome Atlas Stomach adenocarcinoma; TCGA STAD) according to one example of the present invention.
FIG. 5 shows the results of performing Kaplan-Meier survival analysis of a SFRP4-high group, a SFRP4-low group, a SMC-low patient group, and a SMC-high patient group according to one example of the present invention.
FIG. 6 shows the results of comparing SMC score as a function of cancer stage according to one example of the present invention.
FIG. 7 shows a scatter plot for GC cell lines, cluster A (HS746T and MKN1 cell lines) and cluster B (SNU601 and NCIN87 cell lines), created based on the expression profiles of differentially expressed genes (DEGs) via principal component analysis (PCA) according to one example of the present invention.
FIG. 8a shows the results of gene set enrichment analysis (GSEA) of gastric cancer (GC tumors and GC cell lines) and the results of comparing the SMC scores of clusters according to one example of the present invention.
FIG. 8b is a heatmap showing RNA-sequencing data analyzed in various gastric cancer cell lines according to one example of the present invention.
FIG. 9 shows results indicating that stem-like subtypes may be distinguished through each of the 13 genes for obtaining the SMC score selected according to one example of the present invention.
FIG. 10 shows a heatmap of SRF motif target genes in an SMC-low patient group and an SMC-high patient group in TCGA STAD according to one example of the present invention.
FIG. 11a shows the results of luciferase reporter assay performed on HS746T and MKN1 cell lines transfected with the TAGLN promoter according to one example of the present invention.
FIG. 11b shows the results of analyzing the expression levels of MYOCD, MRTFA (myocardin-related factor A), and MRTFB (myocardin-related factor B) in an intestinal cell-like non-EMT cell line and a stem cell-like EMT cell line according to one example of the present invention.
FIG. 11c shows the results of analyzing the expression levels of SMC genes by RT-PCR after knocking down MRTFA in HS746T and MKN1 cell lines according to one example of the present invention.
FIG. 12 shows the results of calculating, as log₂ intensity values, the mRNA expression levels of 13 SMC score genes in each cell line and comparing the calculated values, according to one example of the present invention.

### Best Mode

One embodiment of the present invention is directed to a composition for predicting the prognosis of cancer, the composition comprising an agent for measuring the expression level of at least one gene selected from the group consisting of TAGLN, CALD1, MYL9, MYLK, ACTA2, TPM2, CNN1, MSN, CNN3, MYH10, MYH11, CRIP2, and ITGA5, or a protein encoded thereby.

Another embodiment of the present invention is directed to a kit for predicting the prognosis of cancer, the kit comprising the composition for predicting the prognosis according to the present invention.

Still another embodiment of the present invention is directed to a method of providing information for predicting the prognosis of cancer, the method comprising a step of measuring the expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a biological sample isolated from a subject of interest that has or is highly likely to develop cancer.

Yet another embodiment of the present invention is directed to a diagnostic system for predicting the prognosis of cancer, the diagnostic system comprising: (a) a measurement unit configured to measure the expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a biological sample isolated from a subject of interest; and (b) a calculation unit configured to calculate a smooth muscle cell (SMC) score from the expression level of the gene or the protein encoded thereby, measured in the measurement unit.

### Mode for Invention

Hereinafter, the present invention will be described in more detail with reference to examples. These examples are only for illustrating the present invention in more detail, and it will be apparent to those skilled in the art that the scope of the present invention according to the subject matter of the present invention is not limited by these examples.

### Preparation Example 1: Data collection from gastric cancer patients

The present inventors collected fresh frozen tumor tissues from gastric cancer patients who underwent curative intent gastrectomy at the Yonsei Cancer Center (Seoul, Korea) and matched clinical data. All experiments in this study were conducted with the approval of the Institutional Review Board (IRB) of Yonsei University College of Medicine, and all samples were collected after obtaining written consent from patients.

### Preparation Example 1: Cell culture

According to one example of the present invention, MKN1, SNU601 and NCIN87 cell lines were cultured in RPMI1640 containing 10% fetal bovine serum (FBS), 2 mM L-glutamine, 100 U/ml penicillin and 100 µg/ml streptomycin according to the guide of the cell line bank, and the HS746T cell line was also cultured in DMEM containing 10% FBS, 2 mM L-glutamine, 100 U/ml penicillin, and 100 µg/ml streptomycin according to the guide of the cell line bank. All cell lines were cultured in an incubator at 37°C under 5% CO₂ and used in experiments after testing for contamination.

### Example 1: Selection of SMC genes

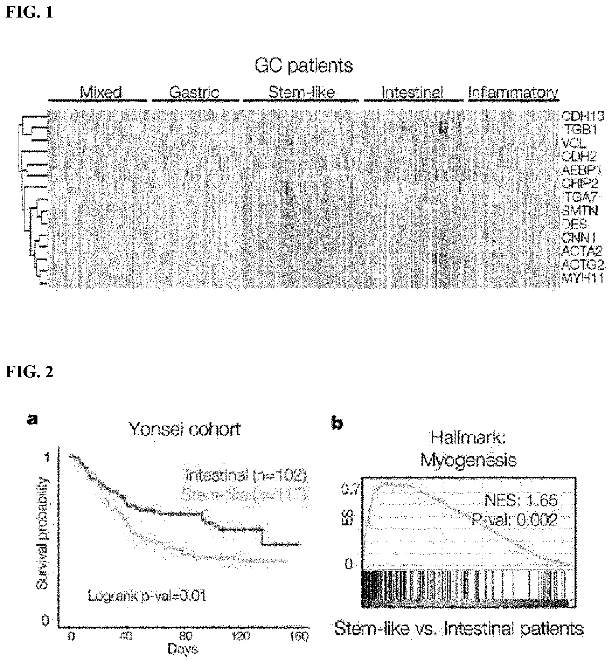

Gastric cancer (GC) patients were divided into five subtypes (mixed, gastric, stem-like, intestinal, and inflammatory) according to a clinically validated classification system. The stem-like subtype showed the worst prognosis, and the transcriptome of the samples from Yonsei cohort gastric cancer patients (n = 497) obtained in Preparation Example 1 were analyzed to investigate the molecular characteristics of stem-like cancer cells. FIG. 1 shows the results of comparing the expression levels of SMC genes between subtypes: mixed (n = 99), gastric (n = 89), stem-like (n = 117), intestinal (n = 102), and inflammatory (n = 90). FIG. 2 shows the survival probability and gene set enrichment analysis (GSEA) results of stem-like GC subtype patients (n = 117) versus intestinal GC subtype patients (n = 102).

As a result, interestingly, it could be confirmed that stem-like GC subtype patients (n = 117) with low survival probability showed higher expression of smooth muscle-specific genes than intestinal GC subtype patients (n = 102) showing substantially different genetic and molecular characteristics. As shown in FIG. 2, it can be seen that SMC genes are up-regulated in the stem-like GC subtype patient group with poor survival probability (see FIG. 1).

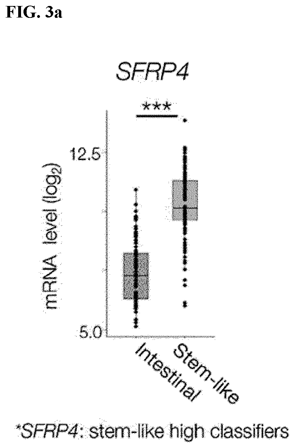

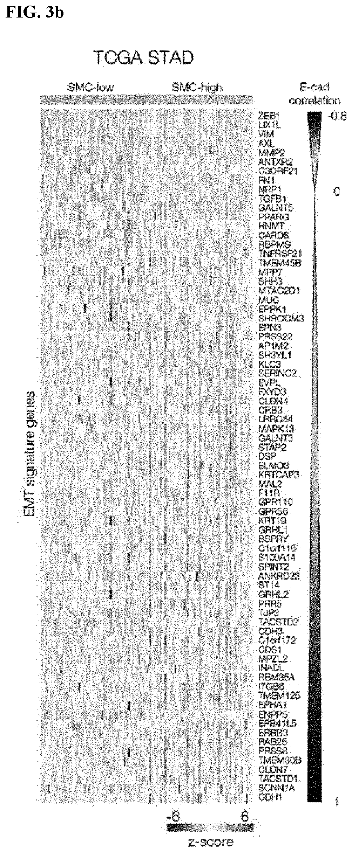

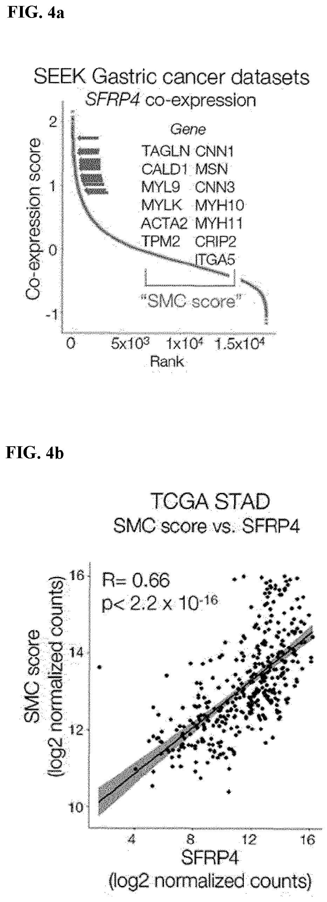

It was verified through a subtyping method that the expression level of the clinical marker SFRP4 was higher in the stem-like group (see FIG. 3a). EMT signature genes in the SMC-low patient group and the SMC-high patient group were heat-mapped (see FIG. 3b). The SFRP4 (secreted frizzled-related protein 4) corresponds to a clinical marker indicating that cancer is malignant or is associated with a poor prognosis that does not respond to standard chemotherapy. In order to select SMC (smooth muscle cell) genes, the present inventors examined the co-expression scores of SFRP4 and SMC genes, thereby selecting TAGLN, CALD1, MYL9, MYLK, ACTA2, TPM2, CNN1, MSN, CNN3, MYH10, MYH11, CRIP2, and ITGA5 (see FIG. 4a). From the above co-expression database, it was confirmed that there was a correlation between SFRP4 expression and SMC markers in 37 human gastric cancer (GC) public datasets. Referring to FIG. 4b, the correlation was confirmed as a result of analyzing the SFRP4 transcript in the gastric tumor dataset from The Cancer Genome Atlas (TCGA).

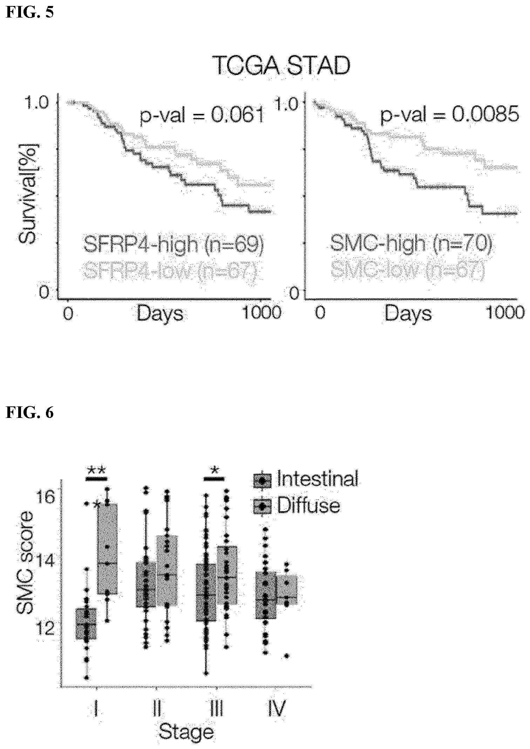

Referring to FIG. 5, as a result of performing Kaplan-Meier survival analysis of a SFRP4-high group, a SFRP4-low group, a SMC-low patient group, and a SMC-high patient group, it was confirmed that the SMC score in the TCGA gastric cancer database more clearly distinguished the patient group with a very poor prognosis from the SFRP4-high group, suggesting that the expression of the SMC genes is related to the malignant prognosis of cancer (see FIG. 5).

To exclude the possibility that tumor invasion into the muscle layer and surrounding soft tissue affects the SMC score, gastric cancer patients were classified by TCGA depending on tumor stage and histological type, and it was confirmed that there was no correlation between the stage at diagnosis and the SMC score. In contrast, the SMC score varied depending on histological type and was higher in diffuse histology (see FIG. 6). Therefore, stem-like GC showed poor prognosis in a stage-independent manner together with upregulation of SMC genes.

### Example 2: Examination of correlation between expression of selected SMC genes and malignant traits

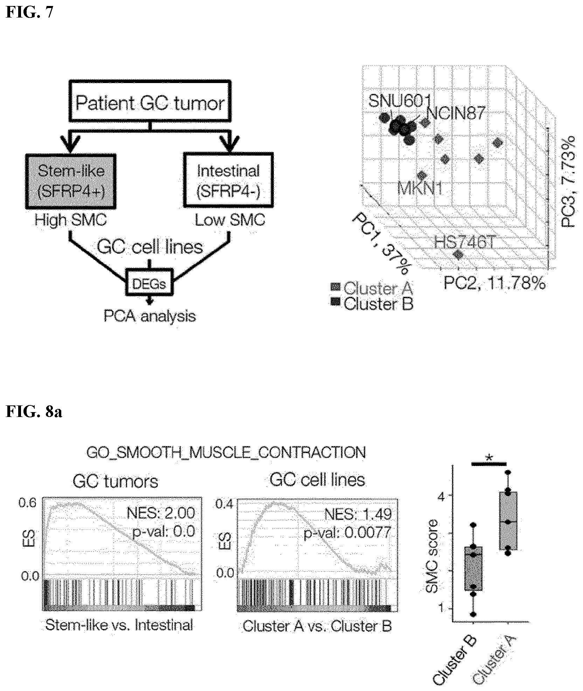

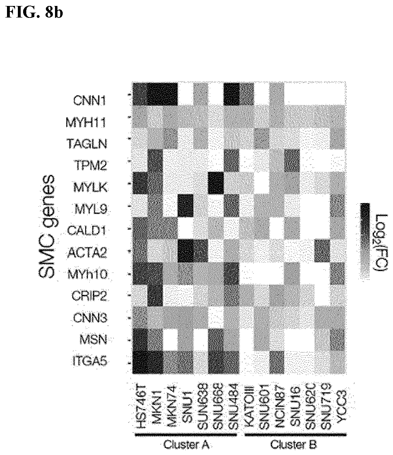

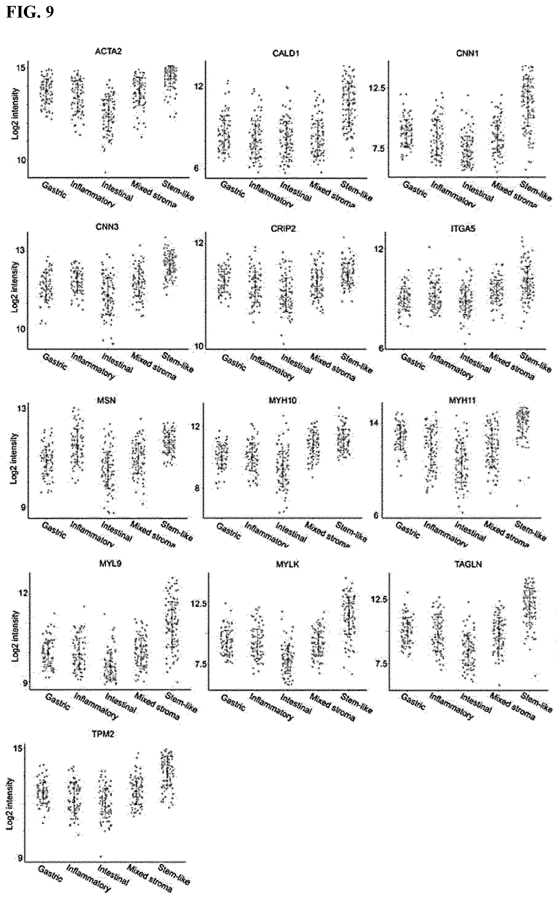

Since bulk analysis of tumor tissue may cause unnecessary contamination by non-cancerous cells, especially stromal muscle cells, which may affect the results of transcriptome analysis, it is important to confirm that expression of the SMC genes is not due to stromal muscle cells and is intrinsic to cancer cells. For this purpose, RNA-seq analysis was performed on gastric cancer cell lines, the cell lines were grouped according to transcriptional characteristics, and a scatter plot of the GC cell lines was created based on the expression profiles of differentially expressed genes (DEGs) upregulated in stem-like patients via principal component analysis (PCA) (see FIG. 7). In gene set enrichment analysis, it could be confirmed that the cell lines of cluster A showed a significant increase in the smooth muscle contraction pathway compared to the cell lines of cluster B, as in GC patients (see FIGS. 8a and 8b). For reference, the cell lines of cluster A are EMT-subtype GC cell lines, and cluster B corresponds to intestinal GC-like non-EMT cell lines. From the above results, it can be seen that there are similar trends between cell lines and patient data, and that there is differentiation between cluster A and cluster B cell lines. That is, since the SMC score is higher in cluster A corresponding to stem-like cells, and the selected SMC genes are highly expressed in cluster A, it can be seen that there is a correlation between the genes and malignant gastric cancer. In addition, it was further verified that the stem-like subtype gastric cancer with the worst prognosis could be distinguished by each of the selected SMC genes (see FIG. 9).

### Example 3: Relationship between MRTFA-SRF transcriptional reprogramming and SMC genes

The transcriptional profiles of GC patients collected from the Yonsei cohort were examined, and SMC genes in smooth muscle were divided into two classes based on whether or not they are dependent on myocardin in the transcription mechanism. Analysis based on hierarchical clustering showed that upregulation of myocardin-dependent SMC genes in stem-like GC was more remarkable than upregulation of myocardin-independent SMC genes. Myocardin-dependent genes were successfully grouped as the mRNA expression levels in stem-like subtype patients increased, whereas myocardin-independent genes did not group stem-like subtype patients and upregulation of the mRNA expression levels thereof also did not appear.

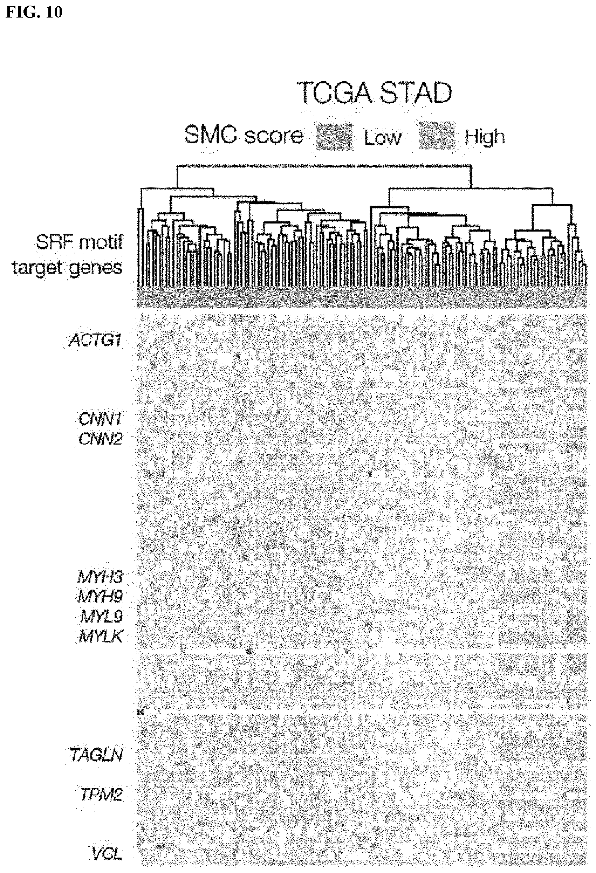

For more detailed information about the importance of myocardin-SRF transcription factor complexes, DNA motif activity analysis was performed with transcriptomes of HS746T, MKN1, KATOIII, and NCIN87 cell lines, and the ISMARA (Integrated System for Motif Activity Response Analysis) algorithm was used to predict regulatory motifs and relevant target genes. According to ISMARA, 106 genes were induced through the CArG box, a DNA motif of SRF10, and most of these genes are involved in actin-related and muscle structure development pathways. SRF motif target genes also include several SMC gene markers, such as ACTG1, CNN1, CNN2, MYH3, MYH9, MYL9, MYLK, TAGLN, TPM2, and VCL, and the expression of SRF motif target genes was confirmed to be upregulated in stem-like GC cell lines. Among GC patients, stem-like patients in the Yonsei cohort and patients with high SMC scores in TCGA were clustered according to SRF motif genes (see FIG. 10).

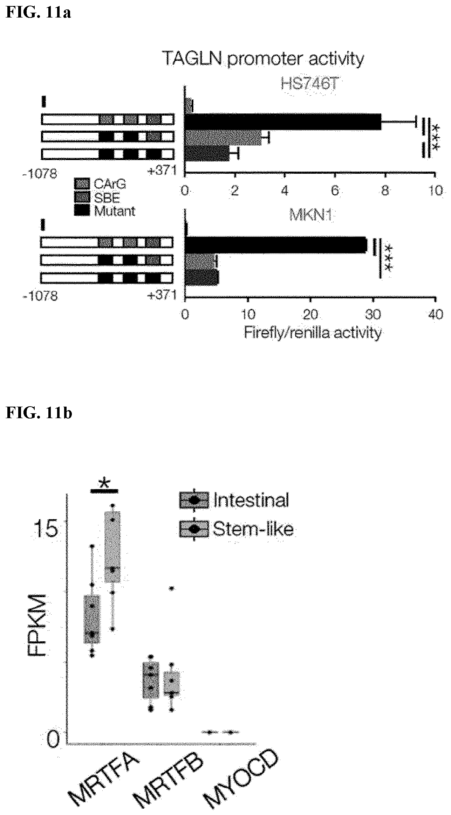

Next, the present inventors mutated two CArG boxes in the transgelin (TAGLN) promoter. For reference, TAGLN, a genetic component for the SMC score, is an actin-binding protein expressed in vascular smooth muscle cells and is also known to be TGFβ-inducible. Luciferase activity assay showed that CArG box ablation significantly inhibited TAGLN promoter activity. However, it was shown that additional ablation of the SMAD binding element (SBE) did not affect activity to that extent, suggesting that the CArG box, but not SBE, is important for the induction of SMC genes in stem-like subtype GC cell lines (see FIG. 11a).

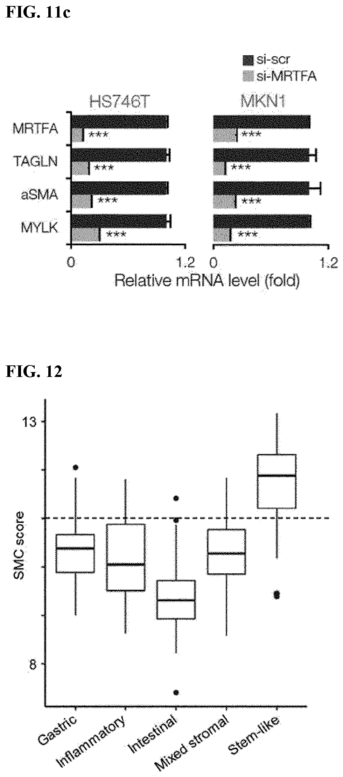

However, the present inventors found that GC cell lines do not express myocardin, which is a master regulator of SMC gene expression in smooth and cardiac muscle cells. Instead, myocardin-related transcription factor-A (MRTFA) and myocardin-related transcription factor-B (MRTFB) as transcriptional coactivators of the myocardin family exist in GC cell lines and have homology to myocardin (see FIG. 11b). In particular, the expression level of MRTFA was higher in stem-like cell lines than in intestinal cell lines, suggesting that MRTFA may be a transcriptional regulator of SMC genes in GC cells (see FIG. 11b). Moreover, it was confirmed that MRTFA knockdown significantly reduced the expression levels of SMC genes (see FIG. 11c).

### Example 4: Determination and verification of SMC score

It was confirmed that gastric cancer with poor prognosis could be diagnosed or predicted based on the SMC score by comparing the SMC scores measured in cluster A (HS746T and MKN1 cell lines) having gastric cancer stem cell properties and cluster B (YCC7 and NCIN87) having general cancer cell properties. Thus, the present inventors verified this confirmation through a patient sample group.

As described in the Preparation Example above, the present inventors collected fresh frozen tumor tissues from gastric cancer patients who underwent curative intent gastrectomy and matched clinical data. Using the collected samples, the present inventors generated two batches of cohort data set (n = 497, GSE13861 and GSE84437), and performed microarray analysis using an Illumina HUmanHT-12 v3.0 Expression BeacChip array. mRNA expression levels were expressed as log intensity values and SMC score was calculated as the average of the log intensities of 13 SMC score genes (TAGLN, CALD1, MYL9, MYLK, ACTA2, TPM2, CNN1, MSN, CNN3, MYH10, MYH11, CRIP2, and ITGA5). It was finally determined that the cutoff value for patients with high SMC scores was 11 (see FIG. 12). This result indicates that it can be effectively predicted that the prognosis of gastric cancer will be very poor when the SMC score according to the present invention is 11 or more.

Taken the above results together, the present inventors found that stem-like subtype GC shows molecular and metabolic characteristics similar to contractile smooth muscle and has a high SMC score, and that there is a correlation between gastric cancer and the SMC score, and the prognosis of gastric cancer is very poor, especially when the SMC score is 11 or more. Through calculation of the SMC score, it is possible to predict the prognosis of malignant gastric cancer based on whether the cancer has acquired SMC properties related to drug resistance, metastasis, survival in metabolic stress, and reduced proliferation.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The composition according to the present invention is capable of predicting the prognosis of cancer, and in particular, may provide information about a cancer with stem cell properties that has a poor prognosis.

Sequence Listing Free Text
SEQ ID NO. 1: TAGLN, Homo sapiens





SEQ ID NO. 2: CALD1, Homo sapiens

MDDFERRREL RRQKREEMRL EAERIAYQRN DDDEEEAARE
RRRRARQERL RQKQEEESLG QVTDQVEVNA QNSVPDEEAK TTTTNTQVEG
DDEAAFLERL ARREERRQKR LQEALERQKE FDPTITDASL SLPSRRMQND
TAENETTEKE EKSESRQERY EIEETETVTK SYQKNDWRDA EENKKEDKEK
EEEEEEKPKR GSIGENQIKD EKIKKDKEPK EEVKSFMDRK KGFTEVKSQN
GEFMTHKLKH TENTFSRPGG RASVDTKEAE GAPQVEAGKR LEELRRRRGE
TESEEFEKLK QKQQEAALEL EELKKKREER RKVLEEEEQR RKQEEADRKL
REEEEKRRLK EEIERRRAEA AEKRQKMPED GLSDDKKPFK CFTPKGSSLK
IEERAEFLNK SVQKSSGVKS THQAAIVSKI DSRLEQYTSA IEGTKSAKPT
KPAASDLPVP AEGVRNIKSM WEKGNVFSSP TAAGTPNKET AGLKVGVSSR
INEWLTKTPD GNKSPAPKPS DLRPGDVSSK RNLWEKQSVD KVTSPTKV
SEQ ID NO. 3: MYL9, Homo sapiens




SEQ ID NO. 4: MYLK, Homo sapiens
MAMISGLSGR KSSTGSPTSP LNAEKLESED VSQAFLEAVA
EEKPHVKPYF SKTIRDLEVV EGSAARFDCK IEGYPDPEVV WFKDDQSIRE
SRHFQIDYDE DGNCSLIISD VCGDDDAKYT CKAVNSLGEA TCTAELIVET
MEEGEGEGEE EEE
SEQ ID NO. 5: ACTA2, Homo sapiens
MCEEEDSTAL VCDNGSGLCK AGFAGDDAPR AVFPSIVGRP
RHQGVMVGMG QKDSYVGDEA QSKRGILTLK YPIEHGIITN WDDMEKIWHH
SFYNELRVAP EEHPTLLTEA PLNPKANREK MTQIMFETFN VPAMYVAIQA

VLSLYASGRT TGIVLDSGDG VTHNVPIYEG YALPHAIMRL DLAGRDLTDY
LMKILTERGY SFVTTAEREI VRDIKEKLCY VALDFENEMA TAASSSSLEK
SYELPDGQVI TIGNERFRCP ETLFQPSFIG MESAGIHETT YNSIMKCDID
IRKDLYANNV LSGGTTMYPG IADRMQKEIT ALAPSTMKIK IIAPPERKYS
VWIGGSILAS LSTFQQMWIS KQEYDEAGPS IVHRKCF
SEQ ID NO. 6: TPM2, Homo sapiens
MDAIKKKMQM LKLDKENAID RAEQAEADKK QAEDRCKQLE
EEQQALQKKL KGTEDEVEKY SESVKEAQEK LEQAEKKATD AEADVASLNR
RIQLVEEELD RAQERLATAL QKLEEAEKAA DESERGMKVI ENRAMKDEEK
MELQEMQLKE AKHIAEDSDR KYEEVARKLV ILEGELERSE ERAEVAESRA
RQLEEELRTM DQALKSLMAS EEEYSTKEDK YEEEIKLLEE KLKEAETRAE
FAERSVAKLE KTIDDLEETL ASAKEENVEI HQTLDQTLLE LNNL
SEQ ID NO. 7: CNN1, Homo sapiens
MSSAHFNRGP AYGLSAEVKN KLAQKYDHQR EQELREWIEG
VTGRRIGNNF MDGLKDGIIL CEFINKLQPG SVKKINESTQ NWHQLENIGN
FIKAITKYGV KPHDIFEAND LFENTNHTQV QSTLLALASM AKTKGNKVNV
GVKYAEKQER KFEPGKLREG RNIIGLQMGT NKFASQQGMT AYGTRRHLYD
PKLGTDQPLD QATISLQMGT NKGASQAGMT APGTKRQIFE PGLGMEHCDT
LNVSLQMGSN KGASQRGMTV YGLPRQVYDP KYCLTPEYPE LGEPAHNHHA
HNYYNSA
SEQ ID NO. 8: MSN, Homo sapiens
GMLREDAVLE YLKIAQDLEM YGVNYFSIKN KKGSELWLGV
DALGLNIYEQ NDRLTPKIGF PWSEIRNISF NDKKFVIKPI DKKAPDFVFY
APRLRINKRI LALCMGNHEL YMRRRKPDTI EVQQMKAQAR EEKHQKQMER
AMLENEKKKR EMAEKEKEKI EREKEELMER LKQIEEQTKK AQQELEEQTR

RALELEQERK RAQSEAEKLA KERQEAEEAK EALLQASRDQ KKTQEQLALE
MAELTARISQ LEMARQKKES EAVEWQQKAQ MVQEDLEKTR AELKTAMSTP
HVAEPAENEQ DEQDENGAEA SADLRADAMA KDRSEEERTT EAEKNERVQK
HLKALTSELA NARDESKKTA NDMIHAENMR LGRDKYKTLR QIRQGNTKQR
IDEFESM
SEQ ID NO. 9: CNN3, Homo sapiens
MTHFNKGPSY GLSAEVKNKI ASKYDHQAEE DLRNWIEEVT
GMSIGPNFQL GLKDGIILCE LINKLQPGSV KKVNESSLNW PQLENIGNFI
KAIQAYGMKP HDIFEANDLF ENGNMTQVQT TLVALAGLAK TKGFHTTIDI
GVKYAEKQTR RFDEGKLKAG QSVIGLQMGT NKCASQAGMT AYGTRRHLYD
PKMQTDKPFD QTTISLQMGT NKGASQAGML APGTRRDIYD QKLTLQPVDN
STISLQMGTN KVASQKGMSV YGLGRQVYDP KYCAAPTEPV IHNGSQGTGT
NGSEISDSDY QAEYPDEYHG EYQDDYPRDY QYSDQGIDY
SEQ ID NO. 10: MYH10, Homo sapiens










































SEQ ID NO. 11: MYH11, Homo sapiens





SEQ ID NO. 12: CRIP2, Homo sapiens
MASKCPKCDK TVYFAEKVSS LGKDWHKFCL KCERCSKTLT
PGGHAEHDGK PFCHKPCYAT LFGPKGVNIG GAGSYIYEKP LAEGPQVTGP
IEVPAARAEE RKASGPPKGP SRASSVTTFT GEPNTCPRCS KKVYFAEKVT
SLGKDWHRPC LRCERCGKTL TPGGHAEHDG QPYCHKPCYG ILFGPKGVNT
GAVGSYIYDR DPEGKVQP
SEQ ID NO. 13: ITGA5, Homo sapiens


























## Claims

1. A composition for predicting prognosis of cancer, the composition comprising an agent for measuring an expression level of at least one gene selected from the group consisting of TAGLN, CALD1, MYL9, MYLK, ACTA2, TPM2, CNN1, MSN, CNN3, MYH10, MYH11, CRIP2, and ITGA5, or a protein encoded thereby.

2. The composition according to claim 1, wherein the agent for measuring the expression level of the gene comprises at least one selected from the group consisting of primers, probes, and antisense nucleotides, which bind specifically to the gene.

3. The composition according to claim 1, wherein the agent for measuring the expression level of the protein comprises at least one selected from the group consisting of antibodies, oligopeptides, ligands, peptide nucleic acids (PNAs), and aptamers, which bind specifically to the protein.

4. The composition according to claim 1, wherein the cancer is a malignant cancer with poor prognosis, which has stem cell properties.

5. The composition according to claim 1, wherein the cancer is at least one selected from the group consisting of gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, and pituitary adenoma.

6. A kit for predicting prognosis of cancer, the kit comprising the composition according to any one of claims 1 to 5.

7. The kit according to claim 6, which is an RT-PCR kit, a DNA chip kit, an ELISA kit, a protein chip kit, a rapid kit, or a multiple reaction monitoring (MRM) kit.

8. A method of providing information for predicting prognosis of cancer, the method comprising a step of measuring an expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a biological sample isolated from a subject of interest that has or is highly likely to develop cancer.

9. The method according to claim 8, wherein the step of measuring the expression level of the gene is performed using an agent comprising at least one selected from the group consisting of primers, probes, and antisense nucleotides, which bind specifically to the gene.

10. The method according to claim 8, wherein the step of measuring the expression level of the protein is performed using an agent comprising at least one selected from the group consisting of antibodies, oligopeptides, ligands, peptide nucleic acids (PNAs), and aptamers, which bind specifically to the protein.

11. The method according to claim 8, wherein the step of measuring the expression level comprises measuring the expression levels of at least two genes selected from the group consisting of TAGLN, CALD1, MYL9, MYLK, ACTA2, TPM2, CNN1, MSN, CNN3, MYH10, MYH11, CRIP2 and ITGA5, or proteins encoded thereby, and
the method further comprises a step of calculating a smooth muscle cell (SMC) score which is an average value of log intensities of the measured expression levels of the genes or the proteins encoded thereby.

12. The method according to claim 11, further comprising a step of predicting that, when the calculated SMC score is 5 to 15 or more, treatment prognosis of the subject of interest is poor, or the survival probability of the subject is low, or the survival time of the subject is short.

13. The method according to claim 11, further comprising a step of predicting that, when the calculated SMC score is less than 5 to 15, treatment prognosis of the subject of interest is good, or the survival probability of the subject is high, or the survival time of the subject is long.

14. The method according to claim 8, wherein the cancer is at least one selected from the group consisting of gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, and pituitary adenoma.

15. A diagnostic system for predicting prognosis of cancer, the diagnostic system comprising:
(a) a measurement unit configured to measure an expression level of at least one gene selected from the group consisting of TAGLN, CALD1, MYL9, MYLK, ACTA2, TPM2, CNN1, MSN, CNN3, MYH10, MYH11, CRIP2 and ITGA5, or a protein encoded thereby, in a biological sample isolated from a subject of interest; and
(b) a calculation unit configured to calculate a smooth muscle cell (SMC) score from the expression level of the gene or the protein encoded thereby, measured in the measurement unit.

16. The diagnostic system according to claim 15, further comprising an output unit configured to output prognosis of cancer in the subject of interest, predicted in the calculation unit.

17. The diagnostic system according to claim 15, wherein the cancer is a malignant cancer with poor prognosis, which has stem cell properties.

18. The diagnostic system according to claim 15, wherein the cancer is at least one selected from the group consisting of gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, and pituitary adenoma.
